# EUROPÄISCHE PATENTSCHRIFT

(11) **EP 0 739 976 B1**
(45) Veröffentlichungstag und Bekanntmachung des Hinweises auf die Patenterteilung: **20.06.2001**
(21) Anmeldenummer: 96106056.3
(22) Anmeldetag: 18.04.1996
(51) Int. Cl.: C11D 3/00, C11D 1/62, C11D 1/52, D06M 13/463, D06M 13/402, A61K 7/50

(54) **Avivagemittel**
Brighteners
Agent d'avivage

(30) Priorität: 28.04.1995 DE 19515646
(43) Veröffentlichungstag der Anmeldung: 30.10.1996
(73) Patentinhaber: Cognis Deutschland GmbH, 40589 Düsseldorf (DE)
(72) Erfinder: Bonastre, Nuria, Dr., 080210 Barberà del Vallès (ES); Pi Subirana, Rafael, Dr., 08400 Granollers (ES)

(56) Entgegenhaltungen:
- EP-A- 0 423 894
- EP-A- 0 634 475
- WO-A-92/18685
- DD-A- 300 605
- DE-A- 4 326 975
- DE-C- 4 335 782

## Beschreibung

Die Erfindung betrifft die Verwendung eines Avivagemittels, enthaltend Esterquats und Fettsaureamide, zur Avivage von Textilien und Haaren.

### Stand der Technik

Quaternierte Ester von Fettsäuren mit Alkanolaminen, sogenannte Esterquats, stellen eine Gruppe von biologisch gut abbaubaren Wäscheweichspülmitteln dar, die in den letzten Jahren zunehmend an Bedeutung gewonnen haben, stellvertretend für den umfangreichen Stand der Technik sei an dieser Stelle auf die Übersichtsartikel von R.Puchta et al. in **Tens.Surf. Det. 30, 186 (1993)** und K.Lagerman in **J.Am.Oil.Chem.Soc. 71, 97 (1994)** verwiesen. Obschon sich die Avivageleistung dieser Stoffe auf einem hohen Niveau befindet, besteht ein ständiges Bedürfnis im Markt, den Weichgriff weiter zu verbessern.

Auch von Fettsäureamiden ist seit langem bekannt, daß sie über avivierende Eigenschaften verfügen [vgl. H.Eckwert in **Seifen-Fette-Anstrichmittel, 74, 527 (1972)].**

Veröffentlichungen aus der jüngeren Vergangeheit, wie z.B. die Druckschriften **DE-A1 4312008, WO 92/00272, WO 92/18685** (Henkel) sowie **WO 92/22535** (Procter & Gamble) zeigen jedoch, daß auch hier der Weichgriff und die Kaltwasserdispergierbarkeit nicht voll zufriedenstellend ist.

Die Aufgabe der Erfindung hat somit darin bestanden, Avivagemittel zur Verfügung zu stellen, die sich durch einen gegenüber Produkten des Marktes verbesserten Weichgriff sowie eine leichtere Kaltwasserdispergierbarkeit auszeichnen.

### Beschreibung der Erfindung

Gegenstand der Erfindung ist die Verwendung eines Avivagemittels, enthaltend
(a) Esterquats und
(b) Fettsäureamide.

Überraschenderweise wurde gefunden, daß Abmischungen der beiden Komponenten zu einer synergistischen Verbesserung des Weichgriffes, eine Verminderung der antistatischen Aufladung von Textilien und Haaren sowie eine Steigerung der Kaltwasserdispergierbarkeit führt. Dies ist umso erstaunlicher, als Abmischungen von Esterquats mit anderen konventionellen Avivagemitteln vom Typ der quartären Ammoniumsalze solche Synergien nicht zeigen.

### Esterquats

Unter der Bezeichnung Esterquats werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quaterniert. Stellvertretend für den umfangreichen Stand der Technik sei an dieser Stelle auf die Druckschriften **US 3915867, US 4370272, EP-A2 0239910, EP-A2 0293955, EP-A2 0295739 und EP-A2 0309052** verwiesen.

Die quaternierten Fettsäuretriethanolaminestersalze folgen der Formel (I) 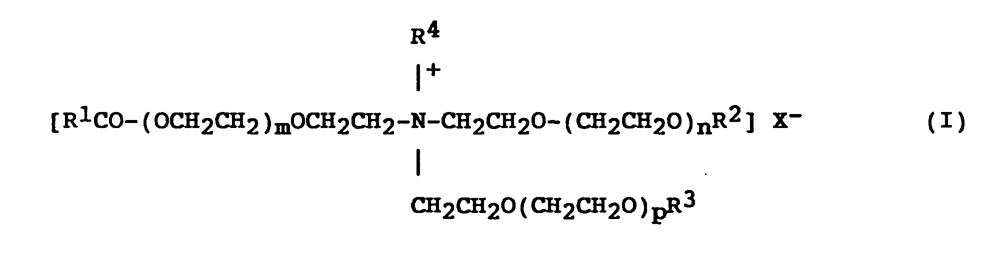 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

Typische Beispiele für Esterquats, die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt.

Zur Herstellung der quaternierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg- bzw. Palmfettsäure (Iodzahl 0 bis 40) ab.

Aus anwendungstechnischer Sicht haben sich quaternierte Fettsäuretriethanolaminestersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht.

Neben den quaternierten Fettsäuretriethanolaminestersalzen kommen als Esterquats ferner auch quaternierte Estersalze von Fettsäuren mit Diethanolalkylaminen der Formel (II) in Betracht, 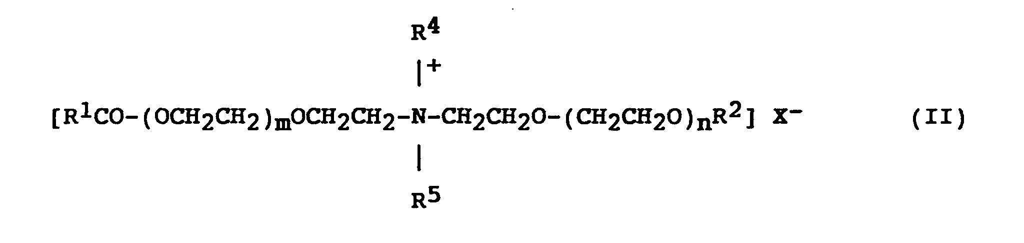 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Als weitere Gruppe geeigneter Esterquats sind schließlich die quaternierten Estersalze von Fettsäuren mit 1,2-Dihydroxypropyldialkylaminen der Formel (III) zu nennen, 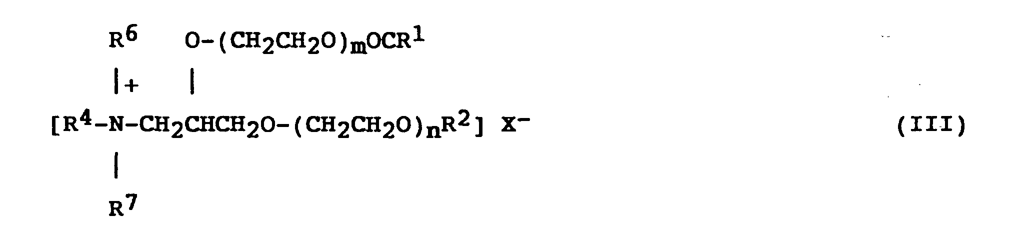 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Hinsichtlich der Auswahl der bevorzugten Fettsäuren und des optimalen Veresterungsgrades gelten die für **(I)** genannten Beispiele auch für die Esterquats der Formeln **(II)** und **(III).** Üblicherweise gelangen die Esterquats in Form 50 bis 90 Gew.-%iger alkoholischer Lösungen in den Handel, die bei Bedarf problemlos mit Wasser verdünnt werden können.

### Fettsäureamide

Fettsäureamide werden durch Kondensation von Fettsäuren mit primären oder sekundären Aminen erhalten. Fettsäureamide, die sich im Sinne der Erfindung als besonders vorteilhaft erwiesen haben, folgen der Formel **(IV),**

**R**^{**8**}**CO-NH-(CH**_{**2**}**)**_{**p**}**NH(CH**_{**2**}**)**_{**q**}**R**^{**9**} **(IV)**

in der R⁸CO für einen linearen, gesättigten Acylrest mit 12 bis 18 Kohlenstoffatomen, R⁹ für eine Hydroxyl- oder Aminogruppe und p und q unabhängig voneinander für Zahlen von 2 bis 4 stehen.

Typische Beispiele sind Kondensationsprodukte von gesättigten Fettsäuren mit 12 bis 18 Kohlenstoffatomen wie Laurinsäure, Myristinsäure, Palmitinsäure, Stearinsäure, Arachinsäure und/ oder Behensäure sowie deren technische Mischungen wie gehärtete Palm- oder Talgfettsäure, mit Aminoethylethanolamin oder Aminoethylethylendiamin. Gleichfalls können technische Mischungen eingesetzt werden, die man durch Umsetzung von gesättigten Tri- bzw. Partialglyceriden der oben angegebenen Fettsäuren mit den entsprechenden Aminen erhält. Das bei dieser Umsetzung freiwerdende Glycerin kann in den Produkten verbleiben, da es die Hydrophilie und die Wiederbenetzbarkeit vorteilhaft beeinflussen kann. Produkte dieser Art sind unter der Bezeichnung Belfasin^{(R)} im Handel erhältlich.

Die erfindungsgemäßen Avivagemittel können die Komponenten (a) und (b) im Gewichtsverhältnis 25 : 75 bis 80 : 20 und vorzugsweise 30 : 70 bis 50 : 50 enthalten.

### Gewerbliche Anwendbarkeit

Die erfindungsgemäßen Mischungen führen zu einer synergistischen Verbesserung des Weichgriffes und reduzieren die statische Aufladung zwischen Fasern unterschiedlichster Beschaffenheit.

Der Gegenstand der Erfindung betrifft daher die Verwendung von Mischungen, enthaltend
(a) Esterquats und
(b) Fettsäureamide
zur Avivage von Textilien und Haaren. Die Mischungen können in Avivagemitteln, Textilsoftenern, Haarkonditioniermitteln und dergleichen in Mengen von 1 bis 50, vorzugsweise 5 bis 35 Gew.-% - bezogen auf die Mittel - eingesetzt werden.

Die Mittel können je nach Einsatzzweck unterschiedliche Hilfs- und Zusatzstoffe enthalten. Hierzu zählen im wesentlichen mit den Esterquats kompatible Tenside wie beispielsweise Fettalkoholpolyglycolether, Alkylpolyglucoside, Fettsäure-N-alkylglucamide, Betaine aber auch Alkylsulfate, Monoglyceridsulfate, Sulfosuccinate und dergleichen.

Im Bereich der Textilavivage können als weitere Hilfs- und Zusatzstoffe beispielsweise schmutzabweisende Polymere, sogenannte "soil reppellants" enthalten. Hierbei handelt es sich vorzugsweise um Ethylenterephthalat- und/oder Polyethylenglycolterephthalatgruppen, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt vorzugsweise im Bereich von 750 bis 5000, d.h., der Ethoxylierungsgrad der Polyethylenglycolgruppenhaltigen Polymere kann ca. 15 bis 100 betragen. Die Polymeren zeichnen sich durch ein durchschnittliches Molekulargewicht von etwa 5000 bis 200.000 aus und können eine Block-, vorzugsweise aber eine Random-Struktur aufweisen.

Bevorzugte Polymere sind solche mit Molverhältnissen Ethylenterephthalat/Polyethylenglycolterephthalat von etwa 65 : 35 bis etwa 90 : 10, vorzugsweise von etwa 70 : 30 bis 80 : 20. Weiterhin bevorzugt sind solche Polymeren, die verknüpfende Polyethylenglycoleinheiten mit einem Molekulargewicht von 750 bis 5000, vorzugsweise von 1000 bis etwa 3000 und ein Molekulargewicht des Polymeren von etwa 10.000 bis etwa 50.000 aufweisen. Beispiele für handelsübliche Polymere sind die Produkte Milease^{(R)} T (ICI) oder Repelotex^{(R)} SRP 3 (Rhone-Poulenc).

Im Bereich der Haaravivage können die Mittel beispielsweise Emulgatoren, Überfettungsmittel, Verdickungsmittel, biogene Wirkstoffe, Filmbildner, Konservierungsmittel, Farb- und Duftstoffe enthalten.

Als **Emulgatoren** kommen sowohl bekannte W/O- als auch O/W-Emulgatoren wie beispielsweise gehärtetes und ethoxyliertes Ricinusöl, Polyglycerinfettsäureester oder Polyglycerinpolyricinoleate in Frage.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen.

Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon, Tenside wie beispielsweise Fettalkoholethoxylate mit eingeengter Homologenverteilung oder Alkyloligoglucoside sowie Elektrolyte wie Kochsalz und Ammoniumchlorid.

Unter **biogenen Wirkstoffen** sind beispielsweise Pflanzenextrakte und Vitaminkomplexe zu verstehen.

Gebräuchliche **Filmbildner** sind beispielsweise Chitosan, mikrokristallines Chitosan, quaterniertes Chitosan, Polyvinylpyrrolidon, Vinylpyrrolidon-Vinyl-acetat-Copolymerisate, Polymere der Acrylsäurereihe, quaternäre Cellulose-Derivate, Kollagen, Hyaluronsäure bzw. deren Salze und ähnliche Verbindungen.

Als **Konservierungsmittel** eignen sich beispielsweise Phenoxyethanol, Formaldehydlösung, Parabene, Pentandiol oder Sorbinsäure.

Als **Perlglanzmittel kommen** beispielsweise Glycoldistearinsäureester wie Ethylenglycoldistearat, aber auch Fettsäuremonoglycolester in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen.

Die folgenden Beispiele sollen den Gegenstand der Erfindung näher erläutern, ohne ihn darauf einzuschränken.

### Beispiele

### I. Eingesetzte Stoffe

A1) Methylquaternierter Dipalmfettsäure-triethanolaminester, Methosulfatsalz
   (Dehyquart^{(R)} AU 56, Henkel Iberica S.A., Barcelona/ES)
B1) Umesterungsprodukt von gehärtetem Palmöl mit Aminoethylethanolamin (Belfasin^{(R)} 43)
B2) Palmfettsäureaminoethylethanolamid
C1) Dimethyldistearylammoniumchlorid Präpagen^{(R)} WK, Hoechst AG, Frankfurt/FRG
C2) 1-Methyl-2-talg-3-talgfettaminoamidoethyl-imidazoliniummethosulfat
   Rewoquat^{(R)} W 7500, Rewo Chemische Werke GmbH, Steinau a.d.Str/FRG

### II. Anwendungstechnische Untersuchungen (nicht erfindungsgemäß)

Durch wiederholtes Waschen gehärtetes Frotteegewebe wurde im Wacker-Gerät mit den Rezepturen nach den Beispielen 1 bis 4 bzw. den Vergleichsbeispielen V1 bis V7 behandelt. Dabei galten folgende Vorgaben:

| | |
|---|---|
| Konzentration | 4 g/l |
| Flottenbeladung | 1 Teil Gewebe / 10 Teile Wasser |
| Wasserhärte | 16 °d |
| Temperatur | 40°C |

Die Beurteilung des Weichgriffs erfolgte subjektiv durch 6 erfahrene Personen, die auf einer Skala von 6 = hart und rauh bis 1 = weich und voluminös Noten vergeben konnten. Die Ergebnisse sind in Tabelle 1 zusammengefaßt (Prozentangaben als Gew.-%):

**Tabelle 1**

| Avivagewirkung | | | | | | | |
|---|---|---|---|---|---|---|---|
| **Bsp.** | **A** | **B** | **C** | **[A] %** | **[B] %** | **[C] %** | **Weichgriff** |
| 1 | A1 | B1 | - | 30 | 70 | - | 1,0 |
| 2 | A1 | B1 | - | 50 | 50 | - | 1,5 |
| 3 | A1 | B1 | - | 75 | 25 | - | 1,5 |
| 4 | A1 | B2 | - | 30 | 70 | - | 1,0 |
| V1 | A1 | - | - | 100 | - | - | 2,0 |
| V2 | - | B2 | - | - | 100 | - | 2,5 |
| V3 | A1 | - | C1 | 30 | 70 | - | 2,5 |
| V4 | - | B2 | C1 | - | 50 | 50 | 3,0 |
| V5 | - | B2 | C2 | - | 50 | 50 | 3,0 |
| V6 | - | - | C1 | - | - | 100 | 3,0 |
| V7 | - | - | C2 | - | - | 100 | 3,0 |

## Patentansprüche

1. Verwendung von Mischungen, enthaltend
(a) Esterquats und
(b) Fettsäureamide
als Haaravivagemittel.

2. Verwendung nach Anspruch 1, **dadurch gekennzeichnet,** daß sie Esterquats der Formel **(I)** enthalten, 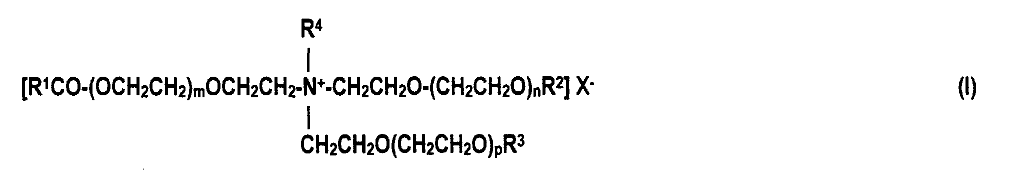 in der R¹CO für einen Acylrest mit 6 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

3. Verwendung nach Anspruch 1, **dadurch gekennzeichnet,** daß sie Esterquats der Formel **(II)** enthalten, 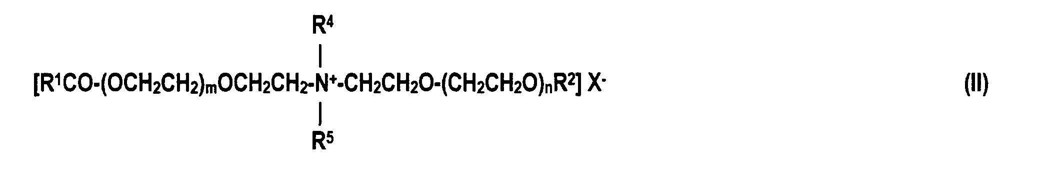 in der R¹CO für einen Acylrest mit 6 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Verwendung nach Anspruch 1, **dadurch gekennzeichnet,** daß sie Esterquats der Formel **(III)** enthalten, 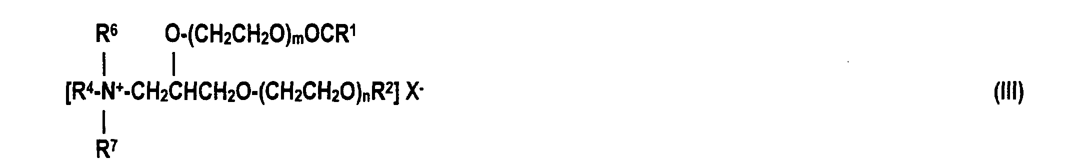 in der R¹CO für einen Acylrest mit 6 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

5. Verwendung nach mindestens einem der Ansprüche 1 bis 4, **dadurch gekennzeichnet,** daß sie Fettsäureamide der Formel **(IV)** enthalten,
**R**^{**8**}**CO-NH-(CH**_{**2**}**)**_{**p**}**NH(CH**_{**2**}**)**_{**q**}**R**^{**9**} **(IV)**
in der R⁸CO für einen linearen, gesättigten Acylrest mit 12 bis 18 Kohlenstoffatomen, R⁹ für eine Hydroxyl- oder Aminogruppe und p und q unabhängig voneinander für Zahlen von 2 bis 4 steht.

6. Verwendung nach mindestens einem der Ansprüche 1 bis 5, **dadurch gekennzeichnet,** daß sie die Komponenten (a) und (b) im Gewichtsverhältnis 25 : 75 bis 80 : 20 enthalten.

## Claims

1. The use of mixtures containing
(a) esterquats and
(b) fatty acid amides
as hair conditioners.

2. The use claimed in claim 1, characterized in that they contain esterquats corresponding to formula **(I):** 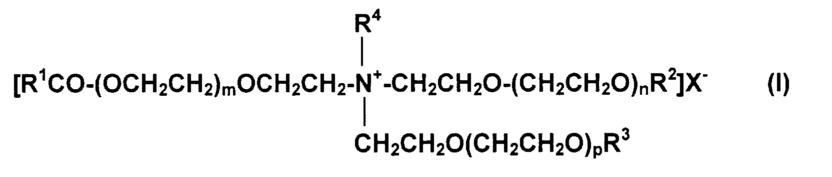 in which R¹CO is an acyl group containing 6 to 22 carbon atoms, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate.

3. The use claimed in claim 1, characterized in that they contain esterquats corresponding to formula **(II):** 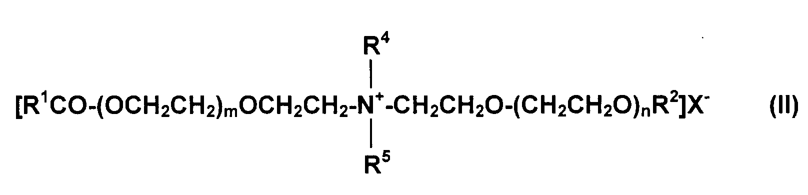 in which R¹CO is an acyl group containing 6 to 22 carbon atoms, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate.

4. The use claimed in claim 1, characterized in that they contain esterquats corresponding to formula **(III):** 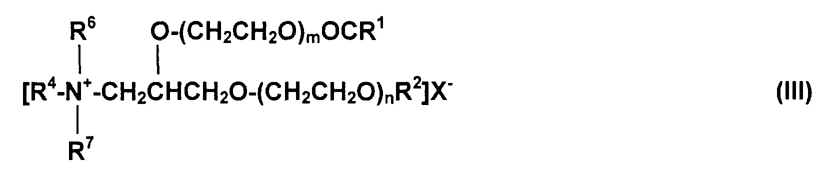 in which R¹CO is an acyl group containing 6 to 22 carbon atoms, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate.

5. The use claimed in at least one of claims 1 to 4, characterized in that they contain fatty acid amides corresponding to formula **(IV):**
**R**^{**8**}**CO-NH-(CH**_{**2**}**)**_{**p**}**NH(CH**_{**2**}**)**_{**q**}**R**^{**9**} **(IV)**
in which R⁸CO is a linear saturated acyl group containing 12 to 18 carbon atoms, R⁹ is a hydroxyl or amino group and p and q independently of one another are numbers of 2 to 4.

6. The use claimed in at least one of claims 1 to 5, characterized in that they contain components (a) and (b) in a ratio by weight of 25:75 to 80:20.

## Revendications

1. Utilisation d'un mélange contenant
(a) des esterquats et
(b) des acides gras,
comme agents d'avivage pour cheveux.

2. Utilisation selon la revendication 1,
caractérisée en ce que
les mélanges contiennent des esterquats de formule (I) 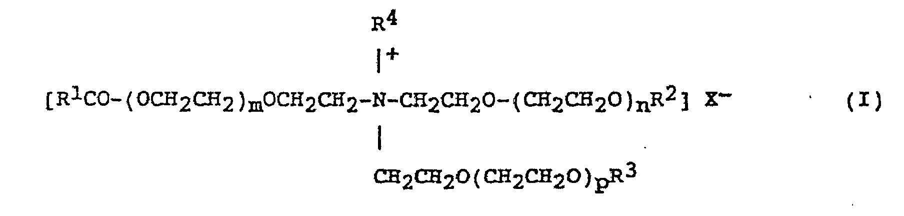 dans laquelle R¹CO représente un radical acyle comportant de 6 à 22 atomes de carbone, R² et R³ représentent indépendamment l'un de l'autre un hydrogène ou R¹CO, R⁴ représente un radical alkyle comportant de 1 à 4 atomes de carbone ou un groupe (CH₂CH₂O)_{q}H, m, n et p valent au total 0 ou des nombres allant de 1 à 12, q représente des nombres allant de 1 à 12 et X représente un halogénure, un sulfate d'alkyle ou un phosphate d'alkyle.

3. Utilisation selon la revendication 1,
caractérisé en ce que
le mélange contient des esterquats de formule (II) 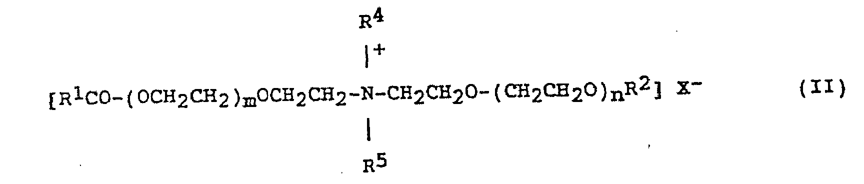 dans laquelle R¹CO représente un₂ radical acyle comportant de 6 à 22 atomes de carbone, R représente un hydrogène ou R¹CO, R⁴ et R⁵ représentent indépendamment l'un de l'autre des radicaux alkyles comportant de 1 à 4 atomes de carbone, m et n valent au total 0 ou des nombres de 1 à 12 et X représente un halogénure, un sulfate d'alkyle ou un phosphate d'alkyle.

4. Utilisation selon la revendication 1,
caractérisé en ce que
le mélange contient des esterquats de formule (III) 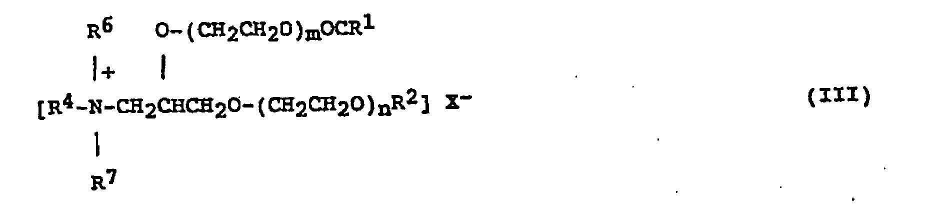 dans laquelle R¹CO représente un radical acyle comportant de 6 à 22 atomes de carbone, R² représente un hydrogène ou R¹CO, R⁴, R⁶ et R⁷ représentent indépendamment l'un de l'autre des radicaux alkyle comportant de 1 à 4 atomes de carbone, m et n valent au total 0 ou des nombres allant de 1 à 12 et X représente un halogénure, un sulfate d'alkyle ou un phosphate d'alkyle.

5. Utilisation selon au moins l'une des revendications 1 à 4,
caractérisée en ce que
les mélanges contiennent des amides d'acides gras de formule (IV)
R⁸CO-NH-(CH₂)ₚNH(CH₂)_{q}R⁹ (IV)
dans laquelle R⁸CO représente un radical acyle linéaire saturé comportant de 12 à 18 atomes de carbone, R⁹ représente un groupe hydroxyle ou amino, et p et q représentent indépendamment l'un de l'autre des nombres allant de 2 à 4.

6. Utilisation selon au moins l'une des revendications 1 à 5,
caractérisée en ce que
les mélanges contiennent les composants (a) et (b) dans un rapport pondéral de 25:75 à 80:20.
